# EUROPEAN PATENT APPLICATION

(11) **EP 3 929 568 A1**
(43) Date of publication of application: **29.12.2021**
(21) Application number: 21180568.4
(22) Date of filing: 21.06.2021
(51) Int. Cl.: G01N 21/64, G01N 35/00, G01N 35/02

(54) **NUCLEIC ACID DETECTION METHOD, NUCLEIC ACID DETECTION DEVICE AND MODULE**

(30) Priority: 26.06.2020 JP 2020110373
(71) Applicant: SYSMEX CORPORATION, Kobe-shi Hyogo 651-0073 (JP)
(72) Inventor: Yotoriyama, Tasuku, Kobe-shi, Hyogo, 651-0073 (JP); Yoshida, Tomokazu, Kobe-shi, Hyogo, 651-0073 (JP); Asano, Kaoru, Kobe-shi, Hyogo, 651-0073 (JP)
(74) Representative: Hoffmann Eitle

(57) **Abstract**

PROBLEM TO BE SOLVED: To provide a nucleic acid detection device capable of processing a large number of samples with a short waiting time, capable of flexibly responding to various examination requirements, and capable of reducing the number of optical detection units.

SOLUTION: A nucleic acid detection device 1 uses a module installation unit 11 capable of installing a plurality of modules 10 capable of accommodating a plurality of tubes 30 containing a sample, a temperature adjusting unit 50 that heats and cools the sample in the tube 30 of each module 10 to the temperature required for nucleic acid amplification, an optical detection unit 12 used commonly by the plurality of modules installed in the module installation unit and which is capable of detecting the amplified nucleic acid of a sample subjected to nucleic acid amplification of a tube 30 by regulating the temperature via the temperature adjusting unit for each module 10 installed in the module installation unit 11, and a moving unit 13 for moving the optical detecting unit and module installation unit relative to each other so as to detect the amplified nucleic acid of the sample in a tube 30 of each of the plurality of modules 10 installed in the module installation unit 11 via the optical detection unit 12.

## Description

### CROSS REFERENCE TO RELATED APPLICATIONS

This application claims priority from prior Japanese Patent Application No. 2020-110373, filed on June 26, 2020, entitled "NUCLEIC ACID DETECTION METHOD, NUCLEIC ACID DETECTION DEVICE AND MODULE", the entire contents of which are incorporated herein by reference.

### FIELD OF THE INVENTION

The present invention relates to a nucleic acid detection method, nucleic acid detection device and module.

### BACKGROUND

In a general PCR (polymerase chain reaction) measurement device, a plate having a large number of wells for accommodating a sample is set in a device equipped with a heating/cooling mechanism, a heating/cooling cycle is repeated to amplify the nucleic acid, and the amplified nucleic acid is optically detected (see, for example, Japanese Patent Application Publication No. 2019-216704).

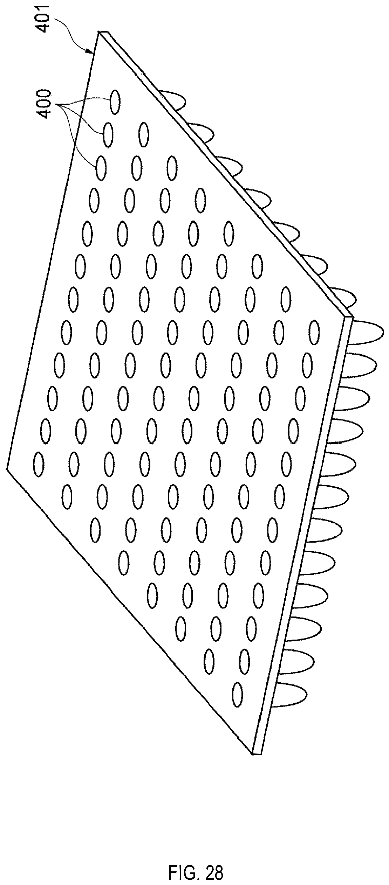

Japanese Patent Application Publication No. 2019-216704 discloses an apparatus for batch processing a large number of samples at one time by using a plate 401 having a large number of wells 400 (for example, a plate of 96 wells) as shown in FIG. 28.

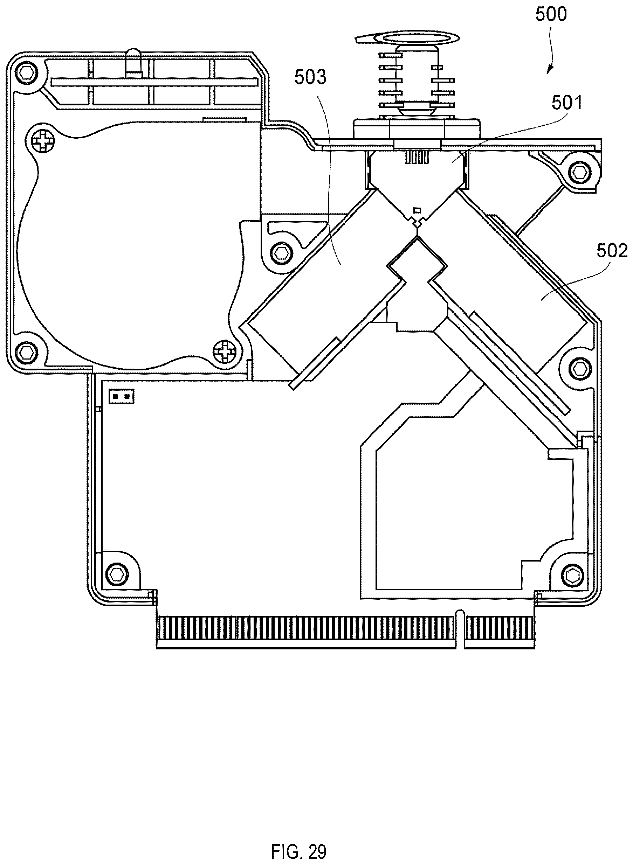

United States Patent No. 6,942,971 discloses an apparatus having a plurality of processing modules, and each processing module independently performs PCR measurement. As shown in FIG. 29, each processing module 500 includes a detector 502 and an LED 503 for detecting the amplified nucleic acid, in addition to a heating/cooling mechanism 501 for a PCR reaction including a reverse transcription reaction and a nucleic acid amplification reaction. Each processing module 500 measures one sample at a time. Since this device includes 16 processing modules 500, PCR measurement can be performed on 16 samples in parallel.

### PRIOR ART DOCUMENTS

### Patent Documents

Patent Document 1: Japanese Unexamined Patent Application Publication No. 2019-216704
Patent Document 2: US Patent No. 6,942,971

### SUMMARY OF THE INVENTION

### PROBLEM TO BE SOLVED BY THE INVENTION

The global epidemic of COVID-19 has increased the demand for PCR testing of infectious viruses. Due to the rapid increase in demand for PCR testing, it is expected that a large number of sample testing requests will occur not only in hospitals but also in places where a large number of people move, such as airports. It is assumed that inspection requests for a large number of samples will be generated one after another, especially at airports, and inspection requests for measuring the samples of passengers whose boarding time is approaching are interrupted by the samples of passengers who arrived earlier.

However, since the measurement device of Japanese Patent Application Publication No. 2019-216704 measures a large number of samples in a batch, the samples arriving during the batch measurement cannot be measured until the batch measurement is completed.

The device of United States Patent No. 6,942,971 uses one optical detection unit for measuring one sample and, hence, a large number of optical detection units are required relative to the number of measurable samples. Since each optical detection unit is expensive, the device is expensive and the also quite large. Since regular maintenance is required for each optical detection unit, the burden on the user is also increased.

The present invention has been developed in view of the above points, and provides a nucleic acid detection method, a nucleic acid detection device, and a module capable of flexibly responding to various inspection requirements while reducing the number of optical detection units.

### MEANS FOR SOLVING THE PROBLEM

As a result of diligent studies on the above-mentioned problems, the present inventors have found that in the above-mentioned apparatus, the optical detection unit is used only for a short time in the reaction cycle of nucleic acid amplification in a series of PCR steps, and at other times is not utilized, hence, the present invention was developed by paying attention to the fact that the optical detection unit is used only for short periods and therefore has a low operating rate. That is, the present invention includes the following aspects.

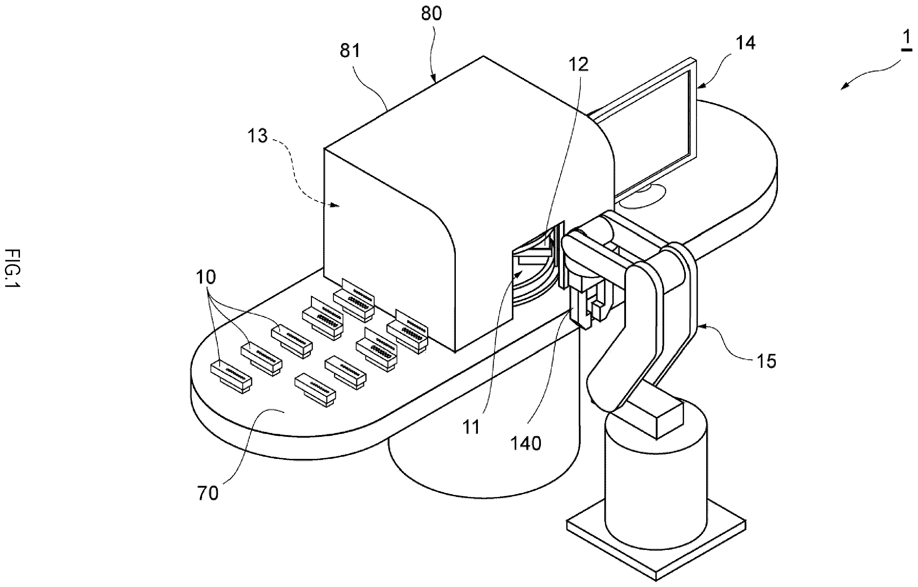

As shown in FIGS. 1, 2, 4, 7, and 27, the nucleic acid detection method of the present invention includes installing a plurality of modules (10) capable of holding a container (30) containing a sample for a nucleic acid amplification reaction in a module installation unit (11); adjusting a temperature of the sample so as to repeat a nucleic acid amplification cycle for each of the plurality of modules (10) installed in the module installation unit (11); moving the module installation unit (11) relative to an optical detection unit (12) shared by the plurality of modules (10), and positioning each of the plurality of modules (10) at a position where the optical detection unit (12) can detect an amplified nucleic acid of the sample; and detecting the amplified nucleic acid of the sample for each of the plurality of modules (30) by the optical detection unit (12).

According to this aspect, since the amplified nucleic acid can be detected in parallel for a plurality of modules, after the processing for one module is completed, the detection for other modules placed in the module installation unit is continued and the next waiting module can be installed in the module installation unit as nucleic acid detection starts. Therefore, it is possible to flexibly respond to various examination requests. According to this aspect, since the optical detection unit (12) is shared by a plurality of modules (10), the number of optical detection units (12) can be reduced relative to the number of samples that can be measured compared with the conventional technique in which one optical detection unit (12) is used to measure one sample and, as a result, the cost of the device can be reduced and the size of the device can be reduced. Since the optical detection unit (12) is shared, the periodic maintenance of the optical detection unit (12) can be reduced, and the burden on the user can be reduced.

As shown in FIGS. 1, 7, 19 and 20, in adjusting temperature, the optical detection unit (12) also may detect the amplified nucleic acid for each of the plurality of modules (10) at a specific timing of the nucleic acid amplification cycle (C) performing the detection a predetermined number of times according to the repetition of the nucleic acid amplification cycle (C), and detecting an amplified nucleic acid in other modules (10) among the plurality of modules between the first detection and the second detection of the amplified nucleic acid for a predetermined module (10) among the plurality of modules.

According to this aspect, the operating rate of the optical detector (12) is increased, and nucleic acid detection of a sample can be performed more efficiently with a smaller number of optical detection units (12).

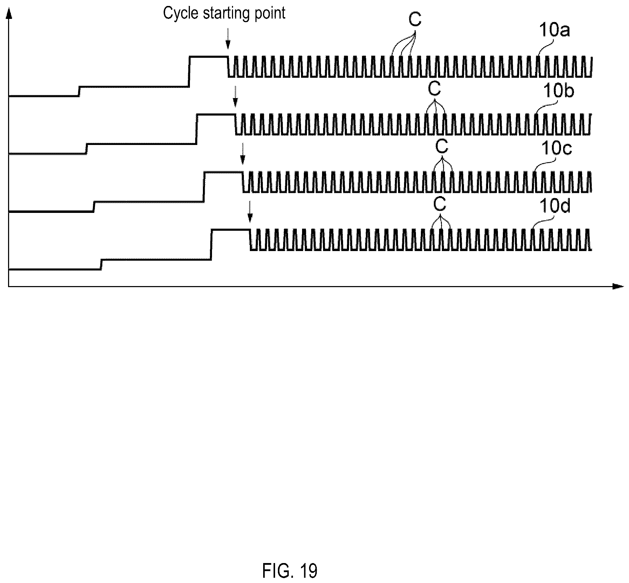

As shown in FIGS. 1 and 19, in adjusting temperature, the temperature may be adjusted so that the starting point of the repeated nucleic acid amplification cycle (C) shifts in each of the plurality of modules (10).

According to this aspect, when a nucleic acid of the sample contained in the container (30) held in each module (10) is detected at a specific timing of the nucleic acid amplification cycle, nucleic acid detection of a sample can be efficiently performed using a shared optical detector (12) for detection.

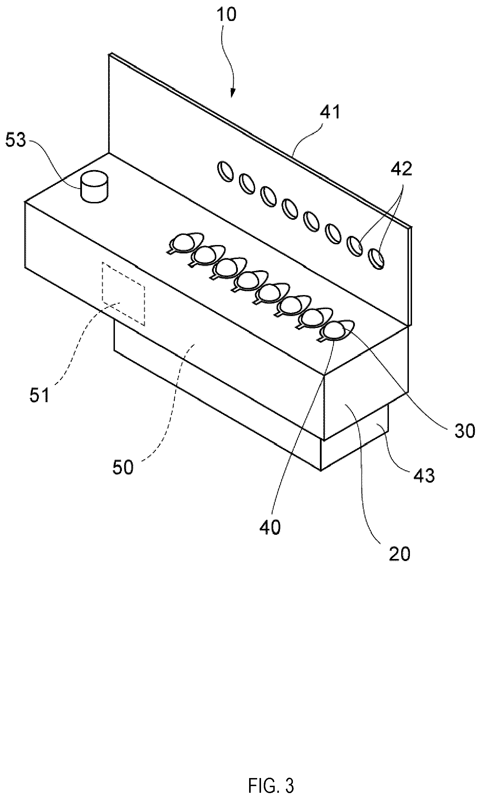

As shown in FIG. 3, temperature adjusting may be performed by a temperature regulation unit (50) provided in each of the plurality of modules (10).

According to this aspect, the temperature can be adjusted at individual timings for each module (10). As a result, the degree of freedom in nucleic acid amplification timing and nucleic acid detection timing of the sample contained in the container (30) held in each of the plurality of modules (10) is increased, and efficient nucleic acid detection can be realized in the nucleic acid detection device (1).

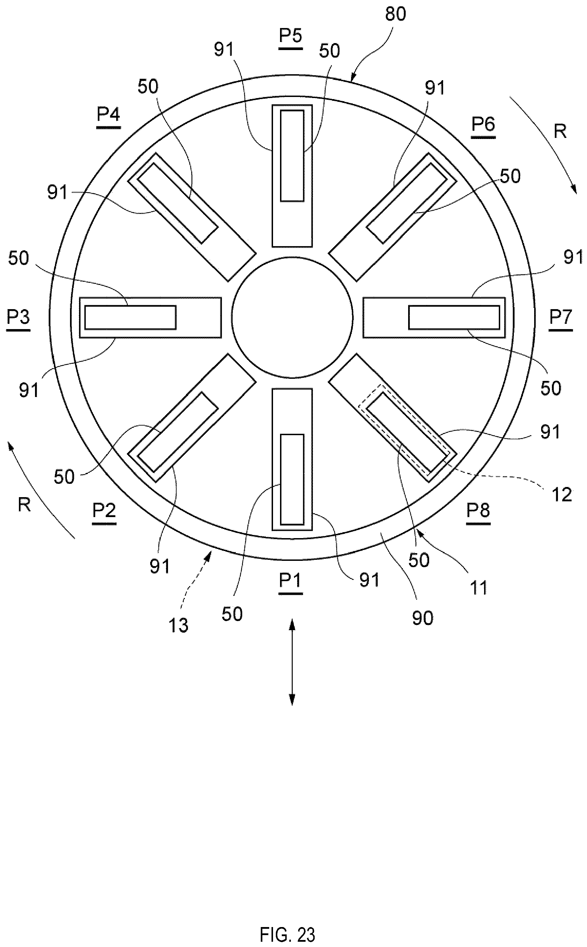

As shown in FIG. 23, temperature adjusting may be performed by the temperature adjusting unit (50) provided in the module installation unit (11). According to this aspect, the degree of freedom in installing the temperature adjusting unit (50) is increased, and the temperature adjusting unit (50) can be easily installed.

As shown in FIG. 1, in temperature adjusting, the temperature of each of the plurality of modules (10) may be raised and lowered to different set temperatures.

According to this aspect, the temperature can be adjusted according to different examination items for each module (10). As a result, nucleic acid detection in different exam items can be efficiently realized for each module (10).

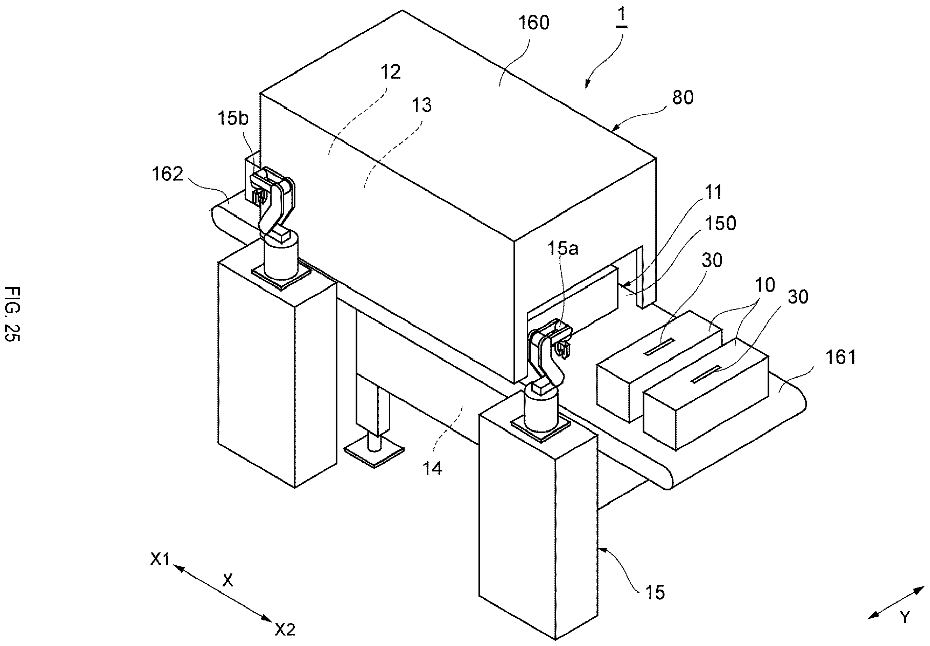

The nucleic acid detection method shown in FIGS. 1 and 25 may further include an installation/removal step for installing each of the plurality of modules (10) in the module installation unit (11) and/or removing each of the plurality of modules (10) from the module installation unit (11) by the transport device (15).

According to this aspect, it is possible to accurately install and remove each of the plurality of modules (10) with respect to the module installation unit (11) in a short time.

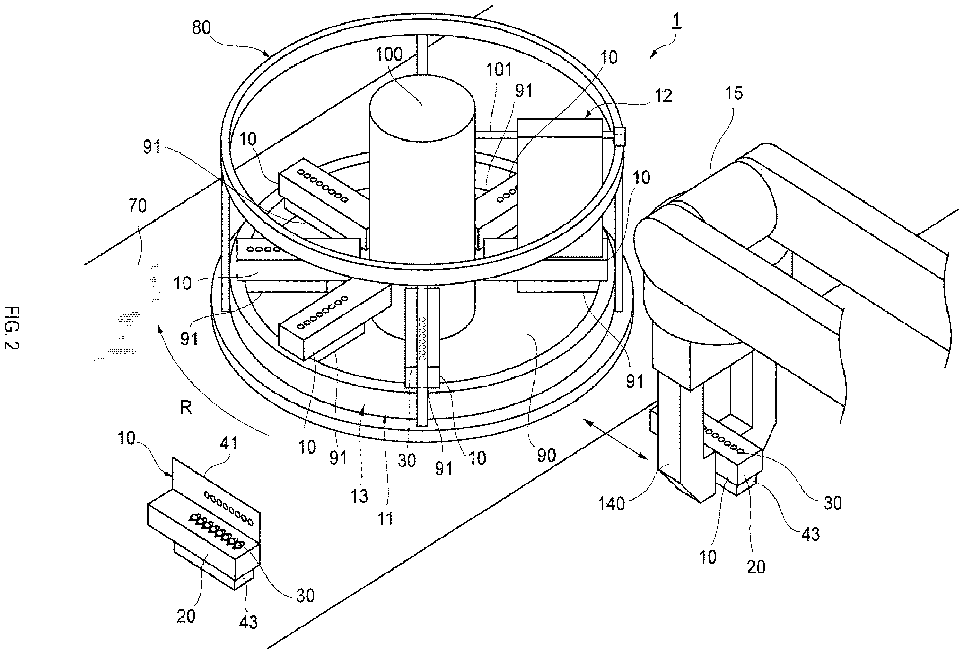

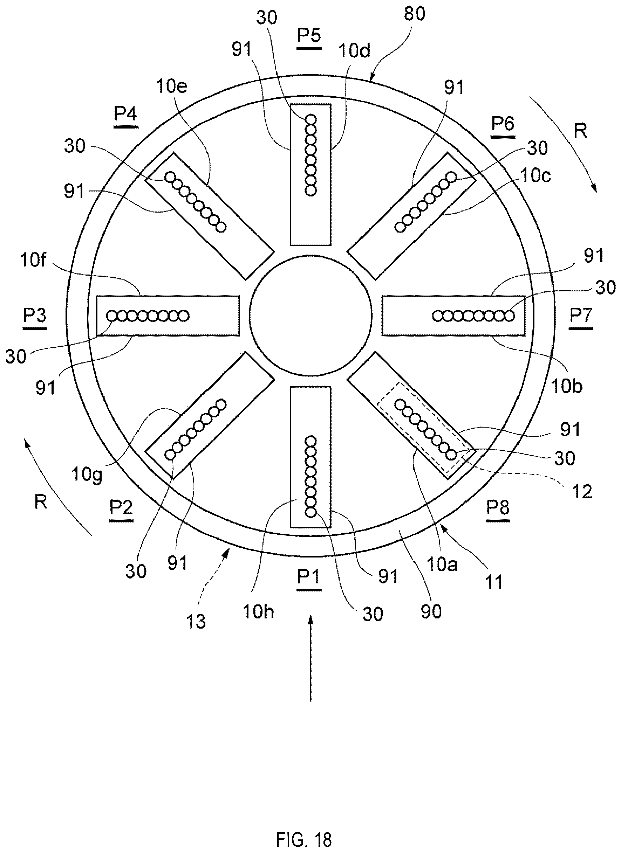

As shown in FIGS. 2 and 18, the module installation unit (11) installs the plurality of modules (10) side by side in the circumferential direction of a circle, and the moving step also may include moving the optical detection unit (12) and the module installation unit (11) relative to one another in the circumferential direction of the circle.

According to this aspect, nucleic acid detection for a plurality of modules (10) can be suitably performed using a shared optical detector (12). Since the module installation unit (11) can arrange the plurality of modules (10) side by side in the circumferential direction of the circle, the module installation unit (11) can be made compact, and the plurality of modules (10) can be efficiently installed and removed.

As shown in FIGS. 25, 26, and 27, the module installation unit (11) also may install the plurality of modules (10) side by side in a linear direction, and the moving step also may include moving the optical detection unit (12) and the module installation unit (11) relative to one another in the linear direction.

According to this aspect, nucleic acid detection for a plurality of modules (10) can be suitably performed using a shared optical detector (12).

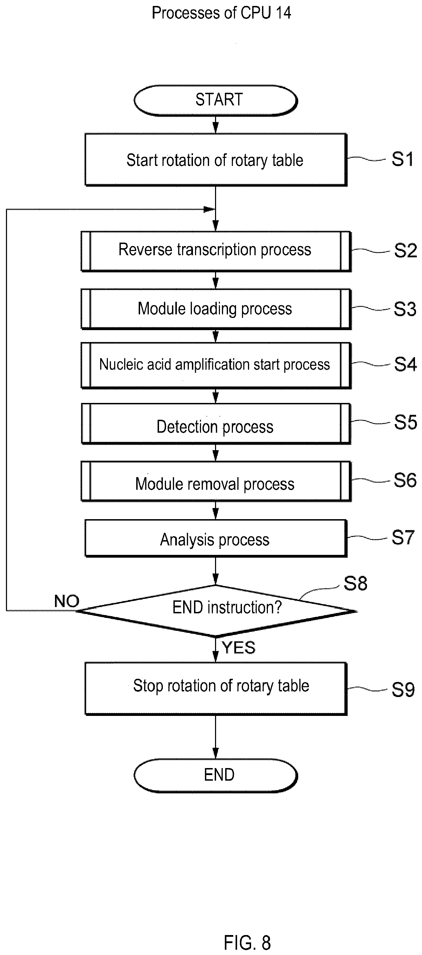

As shown in FIGS. 8, 9 and 14, a reverse transcription reaction of the sample in the container (30) also may be performed before each of the plurality of modules (10) arranged in the module installation unit(11).

According to this aspect, the nucleic acid amplification reaction and the nucleic acid detection of the sample can be immediately performed in the module installation unit (11). A high throughput can be realized since the reverse transcription reaction has already been performed and the nucleic acid detection of a large number of samples can be continuously performed in the module installation unit (11).

As shown in FIGS. 1, 2, 4, 7, 25 and 26, the nucleic acid detection device (1) according to another aspect of the present invention a nucleic acid detection device comprising: multiple modules that can hold a container containing a sample; a module installation unit for installing the plurality of modules; an optical detection unit configured to detect an amplified nucleic acid of the sample contained in the container, and which is shared among the plurality of modules installed in the module installation unit; a moving unit configured to move the optical detection unit and the module installation unit relative to each other so that the optical detection unit can detect the amplified nucleic acid of the sample in the container for each of the plurality of modules installed in the module installation unit; and wherein at least one of the plurality of the modules and the module installation unit includes a temperature adjusting unit for amplifying the nucleic acid of the sample contained in a container.

According to this aspect, since an amplified nucleic acid can be detected in parallel for a plurality of modules, after the processing for one module is completed, the detection for other modules placed in the module installation unit is continued and the next waiting module can be installed in the module installation unit as nucleic acid detection starts. Therefore, it is possible to flexibly respond to various examination requests. According to this aspect, since the optical detection unit (12) is shared by a plurality of modules (10), the number of optical detection units (12) can be reduced relative to the number of samples that can be measured compared with the conventional technique in which one optical detection unit (12) is used to measure one sample and, as a result, the cost of the device can be reduced and the size of the device can be reduced. Since the optical detection unit (12) is shared, the periodic maintenance of the optical detection unit (12) can be reduced, and the burden on the user can be reduced.

As shown in FIGS. 1, 5, 19 and 20, the nucleic acid detection device (1) further includes a control unit (14, 62), and the control unit (14, 62) controls the moving unit (13) and the optical detection unit (12) so as to detect an amplified nucleic acid in another module for each of the plurality of modules at a specific timing, perform detecting the amplified nucleic acid a predetermined number of times according to the repetitions of the nucleic acid amplification cycle, and detect the amplified nucleic acid for another module (10) among the plurality of modules (10) between a first detection and a second detection of the amplified nucleic acid for a predetermined module (10) among the plurality of modules (10).

According to this aspect, the operating rate of the optical detector (12) is increased, and nucleic acid detection of a sample can be performed more efficiently with a smaller number of optical detection units (12).

As shown in FIGS. 1, 5, 19 and 20, the control unit (14, 62) controls the temperature adjusting unit (50) so as to adjust the temperature so that a starting point of the repeated nucleic acid amplification cycle (C) shifts in each of the plurality of modules (10).

According to this aspect, when the nucleic acid detection of each module (10) is performed at a specific timing of the nucleic acid amplification cycle (C), the nucleic acid detection of each module (10) is efficiently performed by using a shared optical detector (12).

As shown in FIG. 3, the temperature adjusting unit (50) also may be provided in the module (10).

According to this aspect, the temperature can be adjusted at individual timings for each module (10). As a result, the degree of freedom in nucleic acid amplification timing and nucleic acid detection timing of the sample of the module (10) is increased, and efficient nucleic acid detection in the nucleic acid detection device (1) can be realized.

As shown in FIG. 23, the temperature adjusting unit (50) also may be provided in the module installation unit (11).

According to this aspect, the degree of freedom in installing the temperature adjusting unit (50) is increased, and the temperature adjusting unit (50) can be easily installed.

As shown in FIGS. 1 and 25, the nucleic acid detection device (1) also includes a transport device (15) installing each of the plurality of modules (10) in the module installation unit (11) and/or removing each of the plurality of modules (10) installed in the module installation unit (11).

According to this aspect, the module (10) can be installed and taken out accurately in a short time with respect to the module installation unit (11).

As shown in FIGS. 8, 9 and 14, the transport device (15) also may install each of the plurality of modules (10) holding a container (30) containing a sample subjected to the reverse transcription reaction in the module installation unit (11).

According to this aspect, nucleic acid amplification and nucleic acid detection of a sample can be immediately performed in the nucleic acid detection device (1). A high throughput can be realized since the reverse transcription reaction has already been performed and the nucleic acid detection of a large number of samples can be continuously performed in the module installation unit (11).

As shown in FIGS. 2 and 18, the module installation unit (11) installs the plurality of modules (10) side by side in the circumferential direction of a circle, and the moving unit 13 also may move the optical detection unit (12) and the module installation unit (11) relative to one another in the circumferential direction of the circle.

According to this aspect, nucleic acid detection for a plurality of modules (10) can be suitably performed using a shared optical detector (12). Since the module installation unit (11) can arrange the plurality of modules (10) side by side in the circumferential direction of the circle, the module installation unit (11) can be made compact, and the plurality of modules (10) can be efficiently installed and removed.

As shown in FIGS. 25, 26, and 27, the module installation unit (11) also may install the plurality of modules (10) side by side in a linear direction, and the moving unit (13) may move the optical detection unit (12) and the module installation unit (11) relative to one another in the linear direction.

According to this aspect, nucleic acid detection for a plurality of modules (10) can be suitably performed using a shared optical detector (12).

As shown in FIGS. 3, 4 and 5, the module (10) of another aspect of the present invention includes a main body (20), an accommodating unit (40) provided on the surface of the main body (20) and capable of accommodating a container (30) containing a sample for nucleic acid amplification, and a temperature adjusting unit (50) provided in the main body (20) for adjusting the temperature of the sample of the container (30) housed in the accommodating unit (40), wherein the temperature adjusting unit (50) includes a heat source (60), a module control unit (62), and a temperature sensor (61).

According to this aspect, the temperature can be appropriately adjusted at a predetermined timing in units of modules (10).

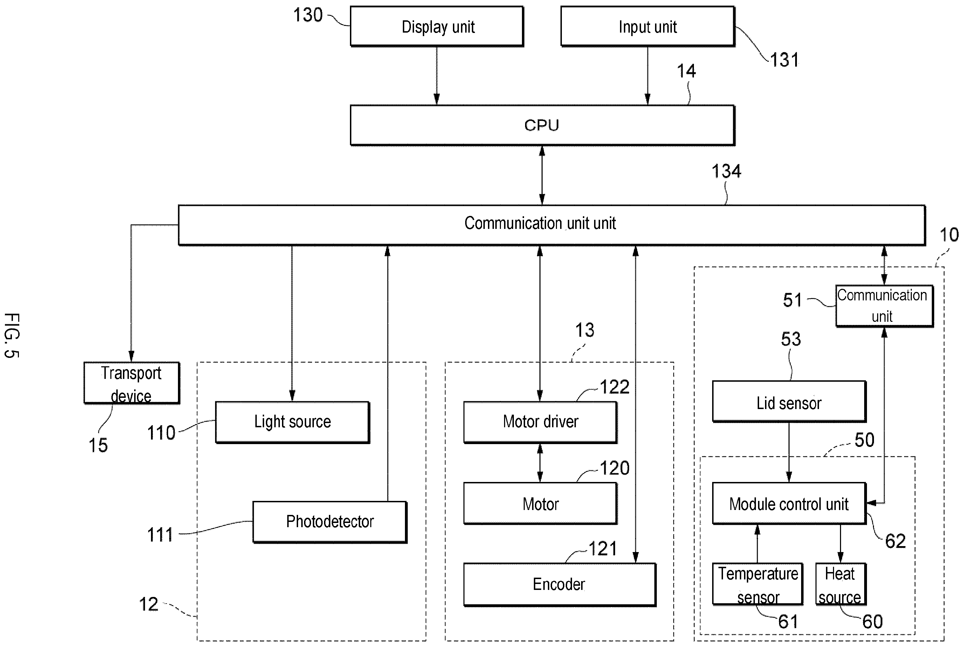

As shown in FIGS. 3 and 5, the module (10) may further include a communication unit (51) for communicating with the outside.

According to this aspect, for example, communication can be performed between the module (10) and the device main body of the nucleic acid detection device (1), and the temperature of the module (10) can be suitably adjusted.

### EFFECTS OF THE INVENTION

The present invention provides a nucleic acid detection method, nucleic acid detection device and module, capable of flexibly responding to various exam requirements while reducing the number of optical detectors, since the measurement of a next sample can be started before the measurement of the entire sample is completed.

### BRIEF DESCRIPTION OF THE DRAWINGS

FIG. 1 is an illustration briefly showing a structure of a nucleic acid detection device according to a first embodiment;
FIG. 2 is an descriptive view showing a nucleic acid detection device with a housing removed;
FIG. 3 is a perspective view of the module;
FIG. 4 is a perspective view of a series of tubes;
FIG. 5 is a block diagram of a device control unit;
FIG. 6 is a descriptive view showing a structure of a device main body as viewed from above;
FIG. 7 is an explanatory diagram showing an internal structure of an optical detector;
FIG. 8 is a flow chart showing an example of a main operation of the device control unit;
FIG. 9 is a flow chart showing an example of detailed operation of the device control unit;
FIG. 10 is a flow chart showing an example of detailed operation of the device control unit;
FIG. 11 is a flow chart showing an example of detailed operation of the device control unit;
FIG. 12 is a flow chart showing an example of detailed operation of the device control unit;
FIG. 13 is a flow chart showing an example of detailed operation of the device control unit;
FIG. 14 is a flow chart showing an example of a main operation of the module;
FIG. 15 is an explanatory diagram showing a state in which a module is installed in a module installation unit;
FIG. 16 is an explanatory diagram showing an example of a nucleic acid amplification cycle;
FIG. 17 is an explanatory diagram showing an optical detection timing in a nucleic acid amplification cycle;
FIG. 18 is an explanatory diagram showing a state in which eight modules are installed in a module installation unit;
FIG. 19 is an explanatory diagram showing nucleic acid amplification cycles of a plurality of modules;
FIG. 20 is an explanatory diagram showing optical detection timing in a nucleic acid amplification cycle of a plurality of modules;
FIG. 21 is an explanatory diagram showing a relationship between a nucleic acid amplification curve based on fluorescence intensity and a threshold value;
FIG. 22 is an explanatory view showing a module installation unit in which 15 modules can be installed;
FIG. 23 is an explanatory diagram showing a structure of a device main body when the temperature adjusting unit is located in the module installation unit;
FIG. 24A is a perspective view briefly showing the structure of a nucleic acid detection device in which a plurality of module installation units are stacked in the height direction, and FIG. 24B is a side view of the nucleic acid detection device;
FIG. 25 is an explanatory diagram briefly showing a structure of a nucleic acid detection device according to a second embodiment;
FIG. 26 is an explanatory view showing a nucleic acid detection device viewed from above;
FIG. 27 is an explanatory view showing a nucleic acid detection device with a housing removed;
FIG. 28 shows a prior art plate; and
FIG. 29 shows a processing module of the prior art.

### DESCRIPTION OF THE EMBODIMENTS OF THE INVENTION

Hereinafter, preferred embodiments of the present invention will be described with reference to the drawings.

In the present embodiment, a nucleic acid detection device that amplifies nucleic acid in a module unit and detects the nucleic acid will be described. The type of sample to which this embodiment can be preferably applied is not particularly limited, but a clinical sample collected from a subject is preferable. In particular, clinical specimens used for examining infectious viruses are preferable. An example of an infectious virus is SARS-CoV-2. Preferred examples of clinical specimens are respiratory-derived specimens such as pharyngeal wipes, nostril wipes, nasal discharge, saliva, sputum and other liquid. Other examples of clinical specimens are whole blood, serum, plasma, cerebrospinal fluid (CSF), pleural effusion, ascites, pericardial fluid, synovial fluid, urine, and stool.

### First Embodiment

As shown in FIG. 1, the nucleic acid detection device 1 includes a plurality of modules 10, a module installation unit 11, an optical detection unit 12, a moving unit 13, a CPU 14, a transport device 15 and the like.

### Module

Module 10 is capable of storing a plurality of containers for containing PCR test samples. As shown in FIGS. 2 and 3, the module 10 has, for example, a rectangular parallelepiped main body 20 having a shape that can be conveyed by the transport device 15. FIG. 2 is an descriptive diagram showing a structure of a nucleic acid detection device 1 in a state in which the housing 81 of the device main body 80, which will be described later, is removed. FIG. 3 is a perspective view of the module 10.

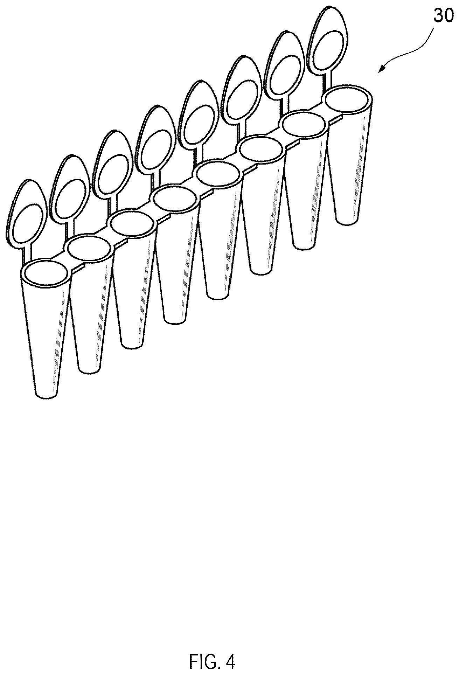

As shown in FIG. 3, for example, a concave receiving unit 40 for accommodating a series of tubes 30 which are a plurality of containers is provided on the upper surface of the main body 20. As shown in FIG. 4, the series of tubes 30 consists of eight containers. The tube 30 is made of a translucent material. The main body 20 is provided with a lid 41 that covers the upper surface thereof. The lid 41 can hold the tube 30 housed in the receiving unit 40 when closed. The lid 41 is formed with a hole 42 for exposing the head of a series of tubes 30. A lid sensor 53 for detecting the opening/closing of the lid 41 is provided on the upper surface of the main body 20. The lid sensor 53 is a sensor that comes into contact with the lid 41 and outputs a detection signal when the lid 41 is closed. For example, a heat sink 43 for quickly dissipating heat is provided on the lower surface of the main body 20. Note that "upper" and "lower" in this specification are based on the posture of the nucleic acid detection apparatus 1 in a normal use state as shown in FIGS. 1 to 3.

As shown in FIGS. 3 and 5, for example, the module 10 includes a temperature adjusting unit 50 and a communication unit 51 for heating and cooling the sample in the tube 30 of the receiving unit 40.

The temperature adjusting unit 50 includes, for example, a heat source 60, a temperature sensor 61, and a module control unit 62, as shown in FIG. 5.

The heat source 60 is, for example, a Peltier element that raises and lowers the temperature by supplying power. The heat source 60 can heat and cool in the temperature range required for the reverse transcription reaction and the nucleic acid amplification reaction of the PCR test. The heat source 60 is provided inside the main body 20.

The temperature sensor 61 is, for example, a thermoelectric body that detects the temperature of a sample in a series of tubes 30 of the receiving unit 40. Note that the temperature sensor 61 also may be a thermistor or a platinum resistance element.

The module control unit 62 is an electronic device that controls the heat source 60 based on, for example, the detection result of the temperature sensor 61. The module control unit 62 is a programmable logic circuit, for example, an FPGA. The module control unit 62 can execute a predetermined program to control the heat source 60 to adjust the temperature of the sample to the temperature required for the reverse transcription reaction and the nucleic acid amplification reaction of the PCR test. In addition, the module control unit 62 can raise or lower the temperature of the sample in a plurality of nucleic acid amplification cycles C described later in the nucleic acid amplification process.

The communication unit 51 is a wireless communication element. The communication unit 51 can send and receive data to and from the CPU 14. The wireless communication method is not particularly limited, but wireless communication via a network such as WIFI may be used, or wireless communication that is not via a network such as Bluetooth (registered trademark) may be used. The module control unit 62 can transmit a signal to or receive a signal from the CPU 14 by communicating with the CPU 14 via the communication unit 51.

A plurality of the above modules 10 are prepared, and each module 10 can accommodate a series of tubes 30 and adjust the temperature of the sample in the tubes 30.

### Device Body

As shown in FIG. 1, the nucleic acid detection device 1 has, for example, a table 70, and the device main body 80 is provided on the table 70. The device main body 80 is covered with, for example, a housing 81. The module installation unit 11, the optical detection unit 12, and the moving unit 13 are provided in the device main body 80. A plurality of modules 10 are arranged on the table 70.

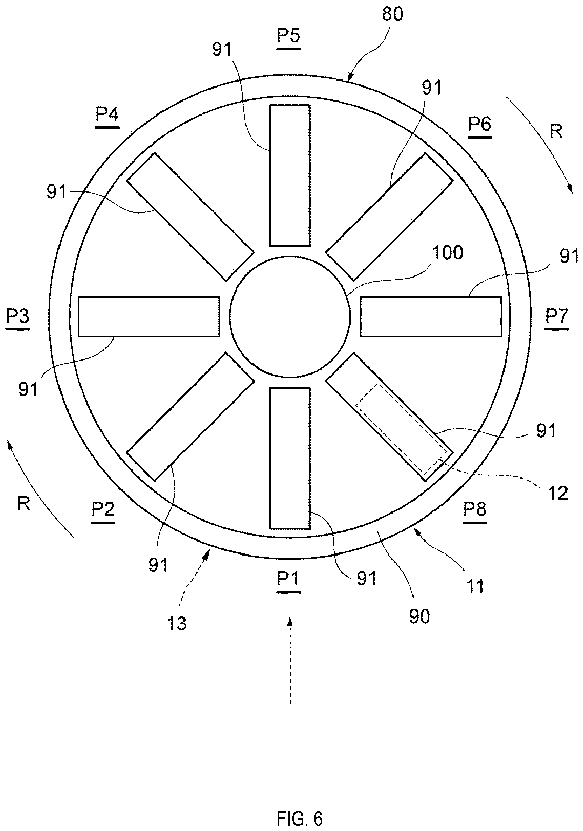

As shown in FIGS. 2 and 6, the module installation unit 11 has, for example, a disk-shaped rotary table 90 and a plurality of, for example, eight module mounting parts 91 arranged on the rotary table 90. Each module mounting part 91 extends radially from the center of the rotary table 90. A module 10, in a state of orientation in which the longitudinal direction of the module 10 faces the radial direction, can be installed in each module mounting part 91. The module mounting parts 91 are arranged at equal intervals in the circumferential direction R of the circle centered on the center of the rotary table 90. That is, the module installation unit 11 includes eight module mounting parts 91 at intervals of 45 degrees in the circumferential direction R. Note that the module mounting part 91 may have a shape such as a groove for positioning the module 10. The module mounting part 91 also may be provided with a terminal for supplying power to the module 10 from the device main body.

Note that in the present embodiment shown in FIG. 6, when the module installation unit 11 is viewed from above, the position of the module mounting part 91, in which the module 10 is carried in and out by the transport device 15, is set as the origin position P1, the position rotated clockwise by 45 degrees is P2 (rotated 45 degrees from the origin position P1), position P3 (rotated 90 degrees from origin position P1), position P4 (rotated 135 degrees from origin position P1), position P5 (rotated 180 degrees from origin position P1), position P6 (rotated 225 degrees from origin position P1), position P7 (rotated 270 degrees from origin position P1), and position P8 (rotated 315 degrees from origin position P1).

As shown in FIGS. 2 and 6, one optical detection 12 is provided on the rotary table 90 of the module installation unit 11. The optical detection unit 12 has a shape extending outward in the radial direction from the center of the rotary table 90, and corresponds to one module mounting part 91 (corresponding to module 10 of the module mounting part 91). When the module mounting part 91 is positioned downward, the optical detection 12 detects fluorescence accompanying nucleic acid amplification of a plurality of samples contained in a plurality of containers of a series of tubes 30 in the module 10 of the module mounting part 91. That is, the optical detection unit 12 can detect fluorescence associated with nucleic acid amplification of a plurality of samples in module units, and is shared by eight modules 10. The optical detection unit 12 is not particularly limited, but is provided at the position P8 (shown in FIG. 6) of the module installation portion 11 when viewed from above.

As shown in FIG. 2, the device main body 80 has, for example, a central pillar 100 located at the center of the rotary table 90, and a support portion 101 extending outward in the radial direction from the upper portion of the central pillar 100. The optical detection unit 12 is supported by the support portion 101 and fixed to the central pillar 100.

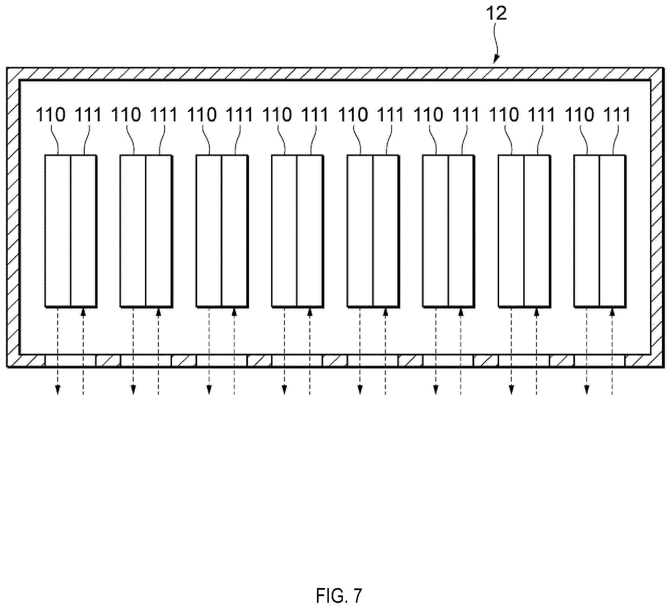

As shown in FIGS. 5 and 7, the optical detection unit 12 includes a light source unit 110 and a photodetector 111. The optical detection unit 12 irradiates a series of tubes 30 with light from the light source unit 110, and the photodetector 111 can detect the fluorescence of the nucleic acid of the sample irradiated by the light. The light source unit 110 includes a plurality of light emitting elements, for example, LEDs arranged in a row along the radial direction of the rotary table 90. Similar to the light source 110, the photodetector 111 includes a plurality of light receiving elements, for example, photodiodes, which are arranged in a row along the radial direction of the rotary table 90. One light emitting element and one light receiving element are arranged in a pair, and the fluorescence generated by the light emitted by the light emitting element is detected by the paired light receiving elements.

The moving unit 13 is provided on the device main body 80 as shown in FIG. 2. The moving unit 13 rotationally drives the rotary table 90 of the module installation unit 11 relative to the optical detection unit 12. As shown in FIG. 5, the moving unit 13 includes, for example, a motor 120 that rotationally drives the rotary table 90, an encoder 121, and a motor driver 122. The encoder 121 detects the rotation angle of the rotary table 90. The motor driver 122 controls the rotation speed and the number of rotations of the motor 120.

The CPU 14 controls the operations of the optical detection unit 12, the moving unit 13, the temperature adjusting unit 50, the transport device 15, and the like. The CPU 14 is connected to the display unit 130, the input unit 131, and the communication unit 134 by a bus. The CPU 14 executes a predetermined program based on the information input from the input unit 131, communicates a control signal with the optical detection unit 12 and the moving unit 13 through the communication unit 134, and controls the operation of the optical detection unit 12 and the moving unit 13. Therefore, for example, the CPU 14 rotates the module installation unit 11 at a predetermined speed by the moving unit 13, causes the light source unit 110 of the optical detection unit 12 to emit light at a predetermined timing, and detects the fluorescence associated with the nucleic acid amplification of the samples in the plurality of containers in the series of tubes 30 by the photodetector 111.

The transport device 15 shown in FIGS. 1 and 2 is, for example, an articulated transport robot, and includes a transport arm 140 that holds and transports the module 10 at its tip. For example, the transport device 15 holds and transports one module 10 from a plurality of modules 10 on the table 70 and mounts the module 10 on the module mounting part 91 of the module installation unit 11, and holds the module 10 in the module mounting part 91 and removed the module 10from the module installation unit 11. For example, the transport device 15 can move the module 10 in and out of the module mounting part 91 at a specific position (origin position P1). The operation of the transport device 15 is controlled by, for example, the CPU 14. A unique ID is assigned to each module 10 arranged on the table 70 in advance, and the user installs the module 10 at a predetermined module installation position according to the ID. By identifying and recognizing the module ID, the transport device 15 holds the specific module 10 at the specific module installation position on the table 70 and mounts it on the module mounting part 91, and returns the module 10 to the original module installation position according to the ID of the module after processing is completed.

### Nucleic Acid Detection Device 1 Operation

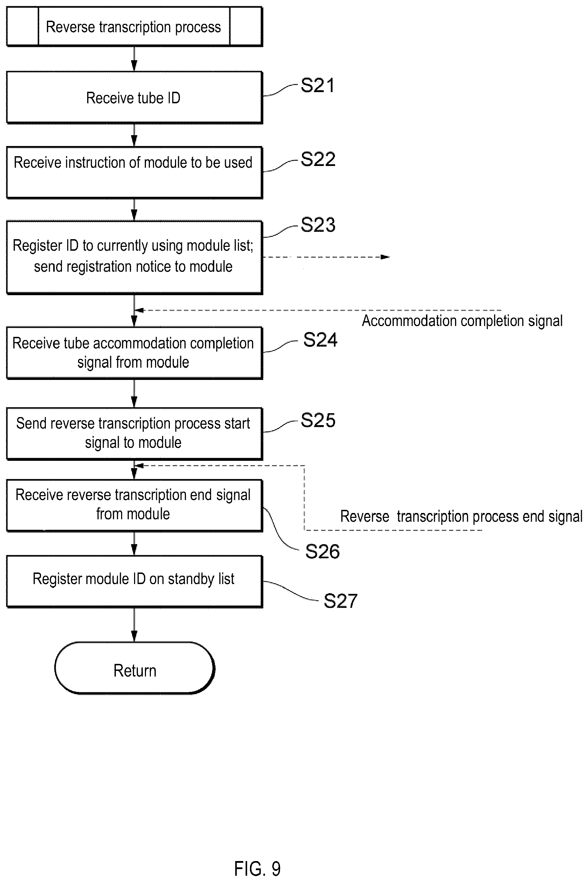

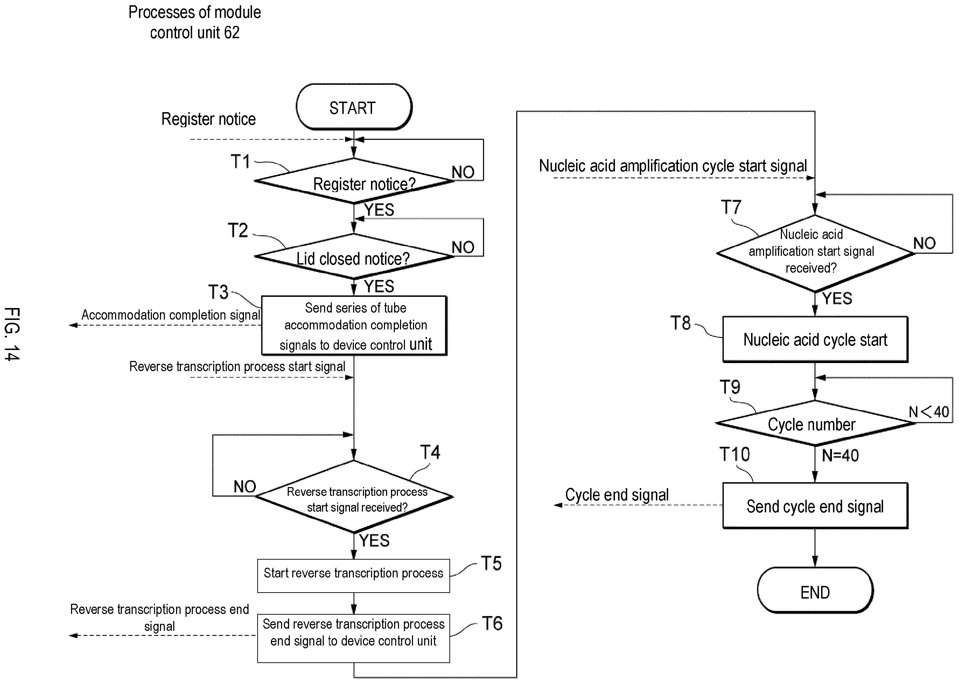

Next, the operation of the nucleic acid detection device 1 will be described. FIG. 8 is a flow chart of a main routine showing an example of a main operation of the CPU 14 in the nucleic acid detection device 1, and FIGS. 9 to 13 are flow charts of subroutines included in FIG. 8. FIG. 14 is a flow chart showing an example of a main operation of the module control unit 62 of the module 10 in the nucleic acid detection device 1.

The nucleic acid detection device 1 mainly performs a reverse transcription process and a nucleic acid amplification process on a sample for a PCR test, and detects the amplified nucleic acid in the nucleic acid amplification process. Hereinafter, an example of the operation of the nucleic acid detection device 1 will be described.

When the nucleic acid detection device 1 is started and the operation of the nucleic acid detection device 1 begins, first, the CPU 14 controls the moving unit 13 to start the rotation of the rotary table 90 of the module installation unit 11 shown in FIG. 2 (S1 of FIG. 8). The rotary table 90 is controlled to rotate 45 degrees at a time to the origin position P1 ,and to pause each time the rotary table 90 rotates 45 degrees. In this way each module mounting part 91 of the rotary table 90 is intermittently rotated so as to temporarily stop at the respective positions P1 to P8. When the rotation of the rotary table 90 is started in S1, intermittent rotation is thereafter performed by 45 degrees according to a predetermined cycle. Hereinafter, the processes S2 to S7 of FIG. 8 are executed in parallel with the periodic rotation of the rotary table 90.

Next, in S2 of FIG. 8, the CPU 14 executes the reverse transcription process. FIG. 9 is a flow chart showing a subroutine of reverse transcription processing. In S21 of FIG. 9, the CPU 14 receives the identification information (ID) of the series of tubes 30 containing the unexamined samples via the input unit 131. The identification information of the tube 30 is manually input by the user through the keyboard provided in the input unit 131, or the machine-readable code attached to the tube 30 is read by a code reader provided in the input unit 131, and sent to the CPU 14. The CPU 14 receives the designation of the module 10 to be used via the input unit 131 (S22 in FIG. 9). Specifically, the user inputs the unique ID assigned in advance to the module 10 via the input unit 131, so that the module ID is input to the CPU 14. At this time, the CPU 14 associates the identification information of the tube 30 with the identification information of the module 10 and stores them in the storage unit. When the CPU 14 receives the designation of the module ID in S22 of FIG. 9, the CPU 14 registers the designated module ID in the in-use module list created in the storage unit, and A registration notification is transmitted to the specific module 10 corresponding to the designated module ID (S23 in FIG. 9).

Next, refer to FIG. 14. In T1 of FIG. 14, the module control unit 62 determines whether the registration notification from the CPU 14 has been received (T1 of FIG. 14). When the module control unit 62 receives the registration notification from the CPU 14 (YES in T1), the module control unit 62 determines whether the lid 41 is closed (T2 in FIG. 14). In S22 of FIG. 9, when a module 10 is designated by the user, a series of tubes 30 containing samples are accommodated in the receiving unit 40 of the designated module 10, and when the lid 41 is closed by the user, the lid sensor 53 detects that the lid is closed and the determination is T2:YES. In T3 of FIG. 14, the module control unit 62 transmits an accommodation completion signal indicating that the accommodation of the tube 30 (accommodation of the sample) is completed to the CPU 14 via the communication unit 51 (T3 of FIG. 14).

Returning to FIG. 9, the CPU 14 receives the accommodation completion signal of the tube 30 from the module 10 (S24 in FIG. 9), and then transmits a signal to start the reverse transcription process to the module 10 (S25 in FIG. 9).

Refer to FIG. 14 once again. The module control unit 62 determines whether the reverse transcription process start signal has been received from the CPU 14 in T4 (T4 in FIG. 14). When the module 10 receives the start signal of the reverse transcription process (YES in T4), the module control unit 62 starts the reverse transcription process (T5 in FIG. 14). In the reverse transcription process, the sample is heated to about 45°C for a certain period of time by the heat source 60. The reverse transcriptase contained in the sample promotes the reverse transcription reaction of the nucleic acid in the sample by heating. When the reverse transcription process is completed, the module control unit 62 transmits a signal indicating that the reverse transcription process is completed via the communication unit 51 to the CPU 14 (T6 in FIG. 14). When the module control unit 62 transmits the reverse transcription process end signal to the CPU 14, the module control unit 62 controls the heat source 60 to heat the sample to a high temperature state of 95°C and maintain the high temperature until the nucleic acid amplification cycle start signal is received from the CPU 14 as described later. In this case, the temperature of the sample at the start of the nucleic acid amplification cycle stabilizes.

Returning to FIG. 9, the CPU 14 receives the reverse transcription process end signal from the module control unit 62 (S26 in FIG. 9). The CPU 14 registers the module ID of the module 10 that has received the reverse transcription process end signal in the standby list created in the storage unit (S27 in FIG. 9). The standby list is a list of modules 10 waiting to be input to the module installation unit 11. When the process of S27 is completed, the CPU 14 returns the process to the main routine of FIG. 8 and proceeds to the process of S3.

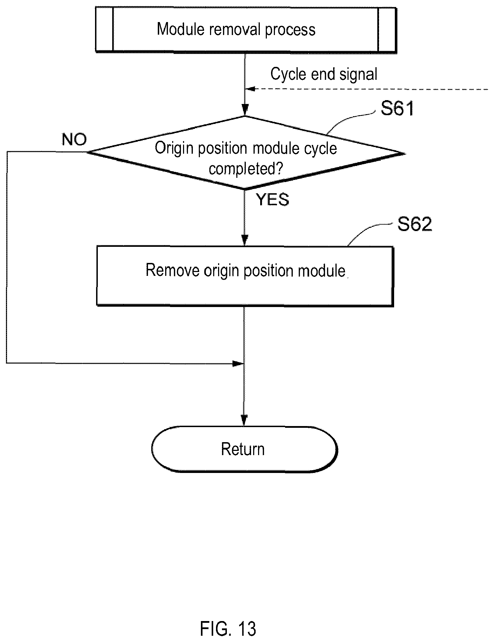

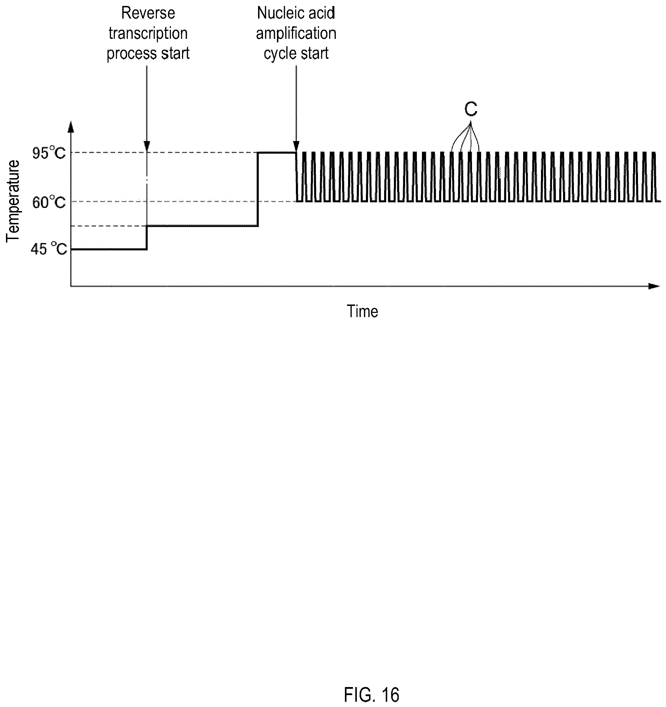

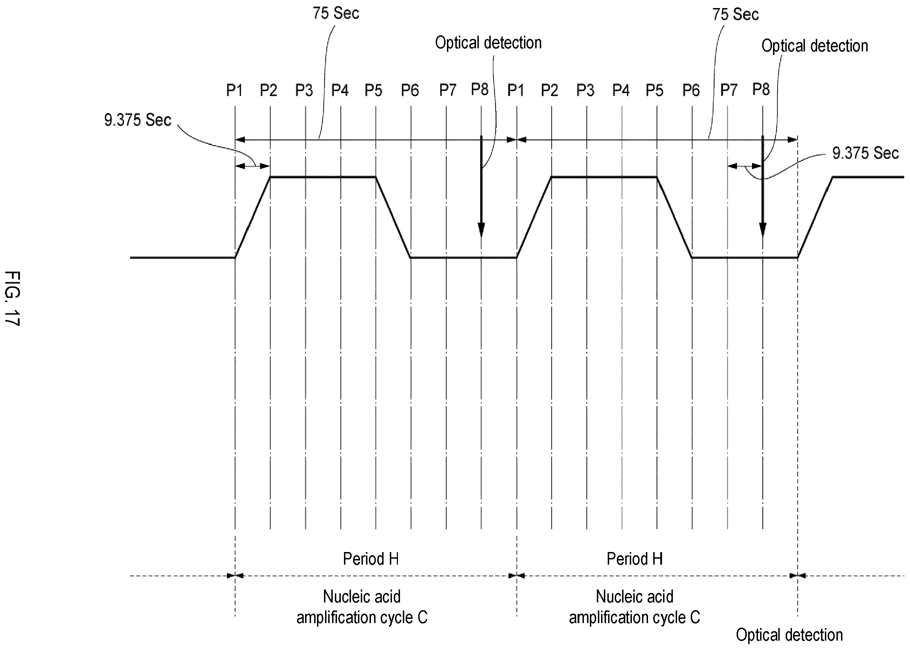

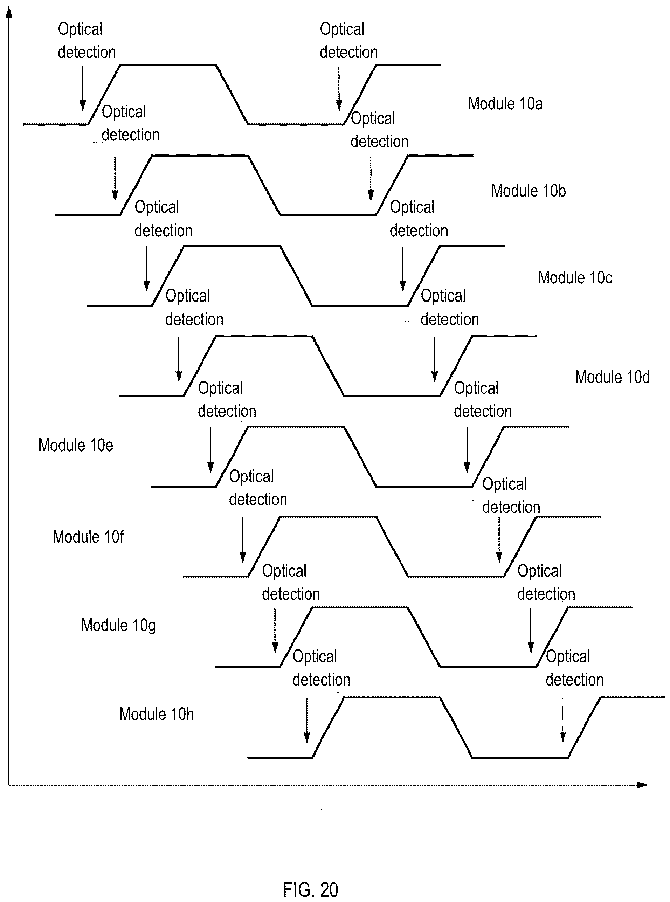

Refer to FIG. 14 once again. Upon receiving the start signal of the nucleic acid amplification cycle from the CPU 14 in T7 of FIG. 14, the module control unit 62 starts the nucleic acid amplification cycle (T8 of FIG. 14). Specifically, the module control unit 62 adjusts the temperature of the sample so as to repeat the nucleic acid amplification cycle C consisting of heating to 95°C and cooling to 60°C as shown in FIG. 16 a predetermined number of times. One nucleic acid amplification cycle C has a cycle of 75 seconds, and the module control unit 62 controls the heat source 60 so as to heat the nucleic acid at a predetermined timing at intervals of 75 seconds and cool it at a predetermined timing according to an internal clock. This nucleic acid amplification cycle C is repeated 40 times, for example. The module control unit 62 counts the number of cycles, and when the number of cycles reaches 40 (YES in T9), the process proceeds to T10 and a cycle end signal is transmitted to the CPU 14 (T10). As shown in FIG. 17, one period H (75 seconds) of the nucleic acid amplification cycle C is matched to the cycle of the rotary table 90 (time required for the rotary table 90 to make one revolution: 75 seconds). That is, the nucleic acid amplification cycle C is performed once in 75 seconds in the module 10 every time the module 10 goes around the central pillar 100 via the rotary table 90 in 75 seconds. The eight module mounting parts 91 on the rotary table 90 are arranged so as to be offset by 45 degrees on the rotary table 90, and the individual module mounting parts 91 make one round in 75 seconds, so that they are 75/8=9.375 second intervals, each module installation unit 91 is sequentially arranged at the origin position P1. As shown in S61 of FIG. 13, when the module 10 is at the origin position P1, the nucleic acid amplification cycle C is started in response to the nucleic acid amplification cycle start signal, and when the module 10 goes around and returns to the origin position P1, the nucleic acid amplification cycle C is controlled to end. Therefore, the nucleic acid amplification cycle C of the module 10 installed in each module mounting unit 91 is phase-shifted by 9.375 seconds (see FIG. 20).

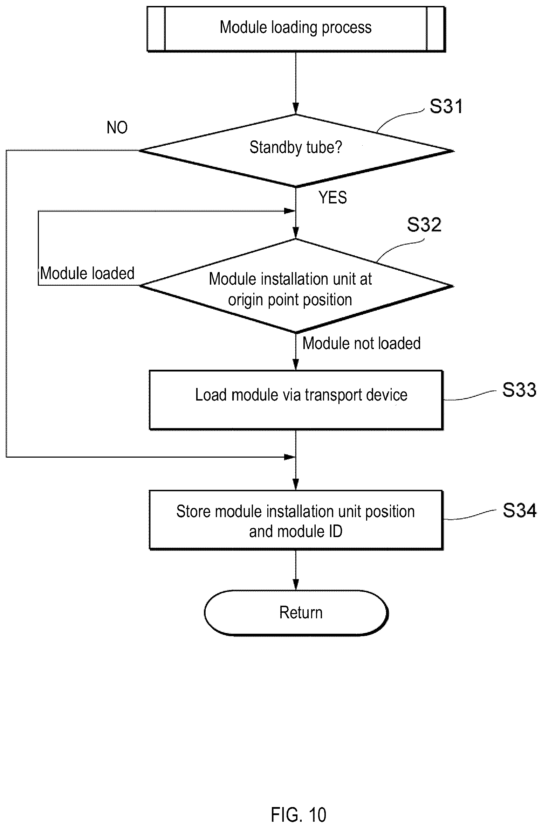

The CPU 14 executes the module loading process in S3 of FIG. 8. Refer to FIG. 10 for the subroutine of the module input process. The CPU 14 determines whether the module ID is registered in the standby list (S31). Next, the CPU 14 confirms whether there is a module in the module mounting part 91 at the origin position P1 of the module installation unit 11 or next in line for the origin position P1 of the module installation unit 11 (S32 in FIG. 10). When there is no module, the CPU 14 transmits a command to the transport device 15, and the transport device 15 holds the module 10 and transfers it to the module mounting part 91 at the origin position P1 (S33 in FIG. 10). The CPU 14 stores the module mounting part 91 and the module IDs in association with each other (S34 in FIG. 10). When the process of S34 is completed, the CPU 14 returns the process to the main routine of FIG. 8 and proceeds to the process of S4.

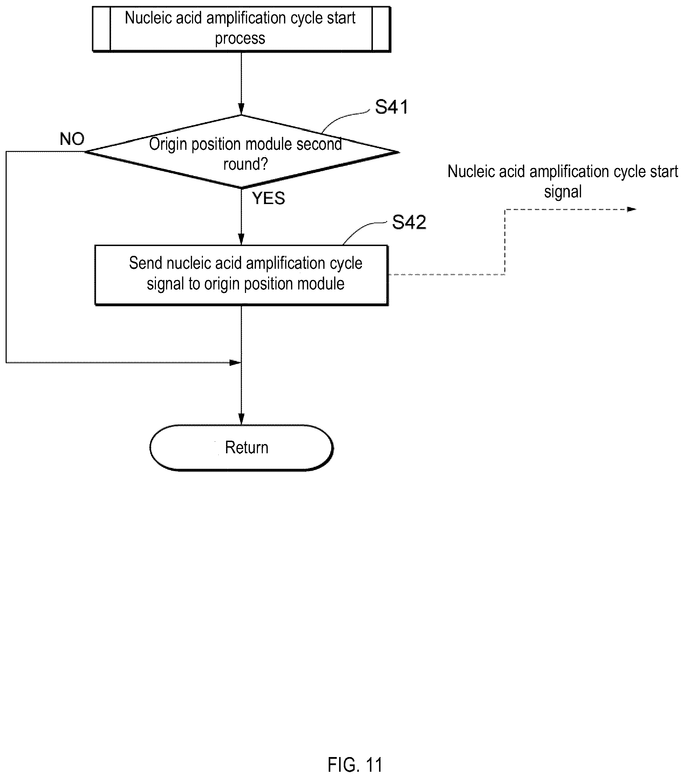

The CPU 14 executes the nucleic acid amplification cycle start processing in S4 of FIG. 8. Refer to FIG. 11 for a subroutine of the nucleic acid amplification cycle start process. The CPU 14 determines whether the module 10 at the origin position P1 is on the second lap (S41). If YES in S41, the CPU 14 transmits a signal instructing the module 10 to start the nucleic acid amplification cycle of the nucleic acid amplification process (S42). Note that the start timing of the nucleic acid amplification cycle is not particularly limited, but may be synchronized with, for example, the timing at which the module 10 starts moving from the origin position P1 to the next position P2. When the CPU 14 finishes the process of S41, the CPU 14 returns the process to the main routine of FIG. 8.

Refer to FIG. 14 once again. Upon receiving the start signal of the nucleic acid amplification cycle from the CPU 14 in T7 of FIG. 14, the module control unit 62 starts the nucleic acid amplification cycle (T8 of FIG. 14). Specifically, the temperature adjusting unit 50 adjusts the temperature of the sample so as to repeat the nucleic acid amplification cycle C consisting of heating to 95°C and cooling to 60°C as shown in FIG. 16 a predetermined number of times. This nucleic acid amplification cycle C is repeated 40 times, for example. The module control unit 62 counts the number of cycles, and when the number of cycles reaches 40 (YES in T9), the process proceeds to T10 and a cycle end signal is transmitted to the CPU 14 (T10). As shown in FIG. 17, the period H of the nucleic acid amplification cycle C is matched to the period of the rotary table 90 (the time required for the rotary table 90 to make one revolution). That is, one nucleic acid amplification cycle C is performed in the module 10 every time the module 10 goes around the central pillar 100 via the rotary table 90. For example, when the module 10 is at the origin position P1, each nucleic acid amplification cycle C is started, and when it goes around and returns to the origin position P1, each nucleic acid amplification cycle C ends.

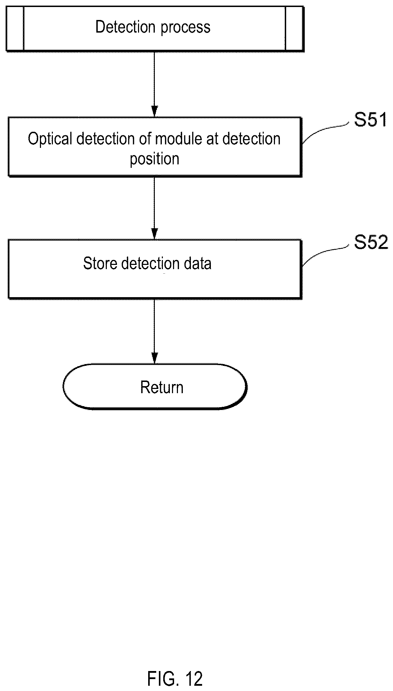

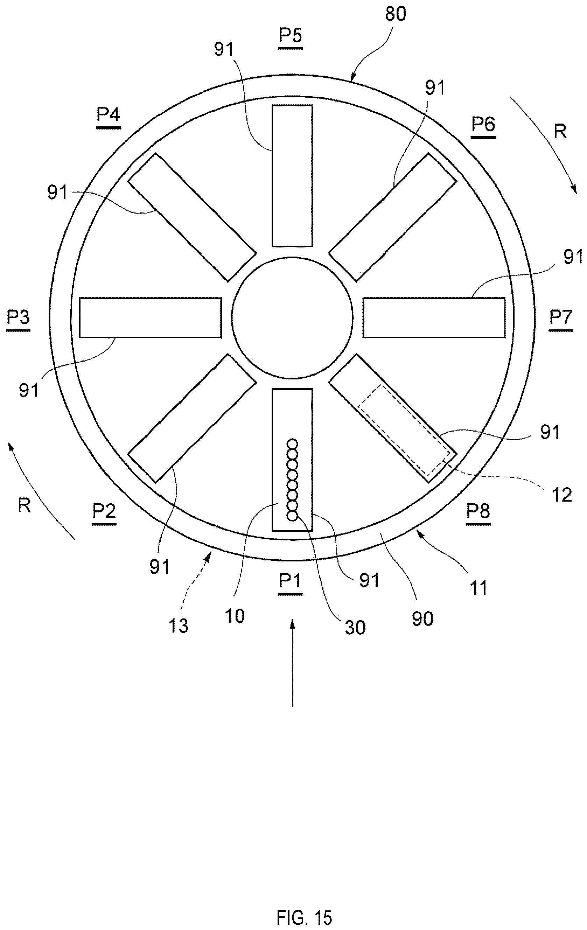

Returning to FIG. 8, when the process of S4 of FIG. 8 is completed, the CPU 14 proceeds to the process of S5 of FIG. 8. The CPU 14 executes the detection process in S5 of FIG. 8. See FIG. 12 for the detection processing subroutine. The CPU 14 detects the fluorescence of the amplified nucleic acid of the sample in the tube 30 of the module 10 when the module 10 is located below the optical detector 12 (when the module 10 is located at the position P8 in FIG. 15) (S51 in FIG. 12). As shown in FIG. 17, the nucleic acid detection by the optical detector 12 is performed at a specific timing in the nucleic acid amplification cycle C, for example, when the sample is cooled to about 60°C. That is, the timing at which the module 10 is located at the position P8 below the optical detector 12 and the specific timing in the nucleic acid amplification cycle C are matched. The CPU 14 stores the detection data based on the fluorescence intensity received from the optical detector 12 in the storage unit (S52 in FIG. 12).

For example, as shown in FIG. 18, a maximum of eight modules 10a, 10b, 10c, 10d, 10e, 10f, 10g, and 10h are sequentially mounted on eight module mounting parts 91, and a nucleic acid amplification process is performed in parallel in each of them. After each of the modules 10a to 10h is mounted on the module mounting part 91 at the origin position P1, the nucleic acid amplification cycle C is started at the timing of one round. As shown in FIG. 19, the starting point of the nucleic acid amplification cycle C for each modules 10a to 10h is shifted between the modules. Since the nucleic acid detection by the optical detector 12 is performed in module units for each turn at the position P8 of the rotary table 90, for example, the detection of other modules 10b to 10h is performed between the first detection and the second detection of one module 10a.

When the process of S52 of FIG. 12 is completed, the CPU 14 returns the process to the main routine of FIG. 8 and proceeds to the process of S6. In S6 of FIG. 8, the CPU 14 executes the module removal process. Refer to FIG. 13 for the subroutine of the module removal process. The CPU 14 determines in S61 whether the module 10 that has arrived or is arriving at the origin position P1 is the module that has transmitted the cycle end signal (S61). If YES in S61, the CPU 14 removes the module 10 from the module mounting part 91 at the timing when the module 10 comes to the origin position P1 (S62 in FIG. 13). Thereafter, a module waiting in the module standby list is then carried into the vacant module mounting part 91. Note that as a matter of course, a module 10 may not be mounted on all of the eight module mounting parts 91. When the process of S62 is completed, the CPU 14 returns the process to the main routine of FIG. 8.

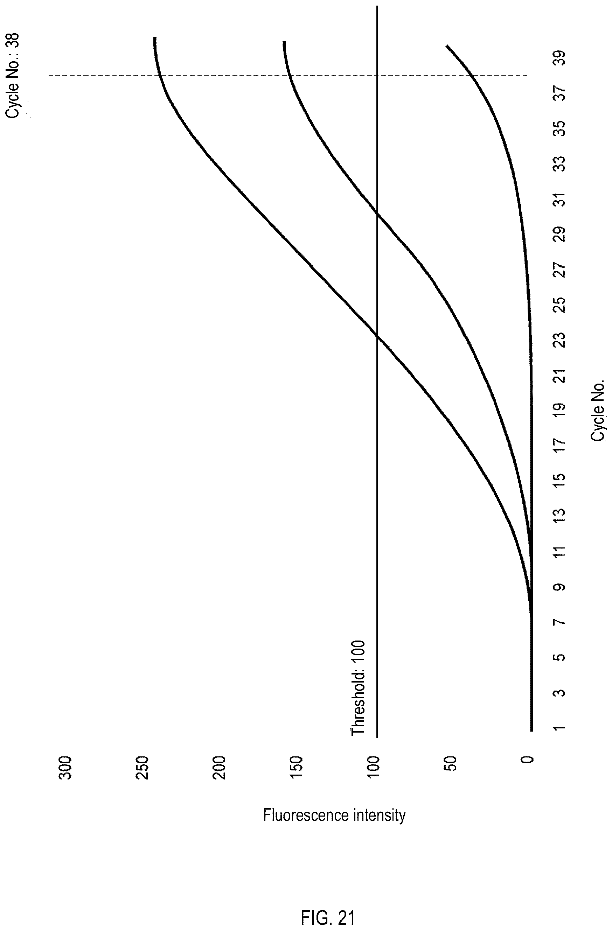

In S7 of FIG. 8, the CPU 14 analyzes the detection data read from each module, creates an amplification curve based on the fluorescence intensity, and acquires the number of cycles (the number of rising cycles) when the fluorescence intensity exceeds a threshold value. FIG. 21 is an explanatory diagram showing the relationship between the amplification curve based on the fluorescence intensity and the threshold value. As shown in the figure, when the nucleic acid amplification cycle is repeated, the fluorescence intensity increases with the nucleic acid amplification in the sample. The rising cycle of fluorescence intensity is faster when the sample contains the target nucleic acid to be detected, and slower when the sample does not contain the target nucleic acid. The CPU 14 sets a flag indicating that the sample is positive when the number of rising cycles is smaller than a predetermined number of cycles, for example, when the number of rising cycles is less than 38 cycles. When the number of rising cycles is 38 cycles or more, the CPU 14 sets a flag indicating that the sample is negative. The CPU 14 stores the detection data and the determination result for each sample in association with the ID of the tube 30 associated with each module 10. In S8 of FIG. 8, the CPU 14 determines whether the end instruction has been received from the user. When it is determined that the end instruction has been received, the CPU 14 proceeds to S9 in FIG. 8, stops the rotation of the rotary table 90 (S9 in FIG. 8), and ends the operation of the nucleic acid detection device 1.

According to the present embodiment, the nucleic acid detection device 1 includes a module installation unit 11 in which a plurality of modules 10 can be installed, a temperature adjusting unit 50, a shared optical detector 12, and a moving unit 13. In this way a plurality of modules 10 accommodating a series of tubes 30 are sequentially installed in the module installation unit 11, the temperature of each module 10 is adjusted for the nucleic acid amplification reaction, and the amplified nucleic acid of the sample can be detected by the optical detector 12 shared among each module 10. As a result, a large number of samples can be processed without waiting time, and various examination requirements can be flexibly met. Further, the number of optical detectors 12 can be reduced, and as a result, the cost of the device can be reduced and the size of the device can be reduced. Since the optical detector 12 is shared, the periodic maintenance of the optical detector 12 can be reduced, and the burden on the user can be reduced.

For example, the CPU 14 and the module control unit 62 control the optical detection unit 12, moving unit 13, and the temperature adjusting unit 50 so as to adjust the temperature of the sample so that the temperature adjusting unit 50 repeats the nucleic acid amplification cycle C consisting of heating and cooling for each module 10, the optical detector 12 detects the amplified nucleic acid at a timing of the nucleic acid amplification cycle C of each module 12, performs detection a predetermined number of times according to the repetition of the nucleic acid amplification cycle C, and detects the amplified nucleic acid of other modules between the first detection and the second detection of the nucleic acid amplification for a predetermined module. In this way the operating rate of the optical detector 12 is increased, and nucleic acid detection of the samples can be performed more efficiently with a smaller number of optical detectors 12. Note that although the above control is realized by the CPU 14 and the module control unit 62 in the present embodiment, the configuration of the control unit is not limited to this, and may be performed by one control unit or by three or more control units.

The CPU 14 and the module control unit 62 control the temperature adjusting unit 50, the moving unit 13, and the like so that the starting point of the repeated nucleic acid amplification cycle C shifts between the modules. In this way the nucleic acid detection of each module 10 can be appropriately performed by using the shared optical detector 12 when the nucleic acid detection of each module 10 is performed at a specific timing of the nucleic acid amplification cycle C, when the nucleic acid detection of each module 10 is performed at a specific timing of the nucleic acid amplification cycle C.

Since the temperature adjusting unit 50 is provided in the module 10, the temperature can be adjusted at individual timings for each module. As a result, the degree of freedom in the timing of nucleic acid amplification and the timing of nucleic acid detection of the sample of the module 10 is increased, and efficient nucleic acid detection can be realized in the nucleic acid detection device 1. Since the reverse transcription process can be performed for each module 10, if a sample is contained in the module 10, the reverse transcription process can be performed first in each module each time, and the nucleic acid amplification process can be waited at an earlier timing. As a result, the nucleic acid amplification process can be efficiently performed in the nucleic acid detection device 1.

Since the nucleic acid detection device 1 includes the transport device 15, the module 10 can be accurately installed and removed from the module installation unit 11 in a short time.

The module installation unit 11 installs a plurality of modules 10 side by side in the circumferential direction R of the circle, and the moving unit 13 relatively moves the optical detector 12 and the module installation unit 11 in the circumferential direction R of the circle. In this way nucleic acid detection for a plurality of modules 10 can be suitably performed using the shared optical detector 12.

Since the module 10 is configured to accommodate 10 or fewer tubes 30, nucleic acid can be detected in small units, and the waiting time for nucleic acid detection of the sample can be reduced.

Since the sample in the tube 30 of the module 10 installed in the module installation unit 11 has undergone a reverse transcription reaction, nucleic acid amplification and nucleic acid detection can be immediately performed in the nucleic acid detection device 1. A high throughput can be realized since the reverse transcription reaction has already been performed and the nucleic acid detection of a large number of samples can be continuously performed in the module installation unit 11.

The module 10 includes a main body 20, an receiving unit 40 provided on the surface of the main body 20 and capable of accommodating the tube 30, a temperature adjusting unit 50 for adjusting the temperature of the sample of the tube 30 accommodated in the receiving unit 40, and the temperature adjusting unit 50 includes a heat source 60, a module control unit 62, and a temperature sensor 61. Therefore, the temperature can be appropriately regulated for each module.

Since the module 10 further includes a communication unit 51 for communicating with the outside, for example, communication can be performed between the module 10 and the CPU 14, and the temperature of the module 10 can be preferentially adjusted.

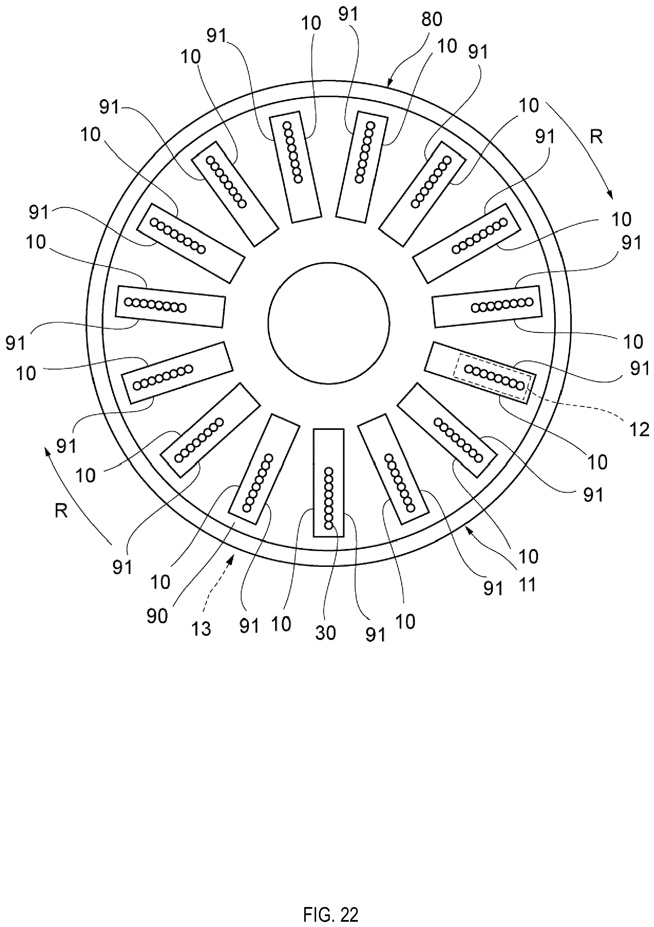

In the above embodiment, the number of module mounting parts 91 of the module installation unit 11 is not limited to eight. For example, as shown in FIG. 22, the number of module mounting parts 91 may be 15. In such a case, the rotary table 90 is intermittently rotated by 24 degrees in order for the transport device 15 to mount the module 10 on each module mounting art 91 and mounts the optical detector 12 detect the nucleic acid of each module 10. In this case, the control of the optical detection unit 12, the moving unit 13, the module 10, and the like may be the same as in the above embodiment.

In the above embodiment, the temperature adjusting unit 50 is provided in the module 10, but it also may be provided in the device main body 80. For example, as shown in FIG. 23, the temperature adjusting unit 50 may be provided in each module mounting part 91 of the rotary table 90. The temperature adjusting unit 50 also may be provided at another position such as the lower part of the rotary table 90 where the sample of the module 10 can be temperature-adjusted in the device main body 80.

Although the transport device 15 carries in and takes out the module relative to the module mounting part 91 at the origin position P1, the module 10 also may be loaded and unloaded to the module mounting part 91 at different positions. The transport device 15 also may include a transport unit for loading in the module 10 and a transport unit for unloading out the module 10.

In the above embodiment, the number of optical detectors 12 is one, but there may be a plurality of optical detectors 12 insofar as they are shared by a plurality of modules 10. Although the optical detector 12 was arranged in the module installation unit 11 at a position P8 deviated from the origin position P1 where the module 10 is loaded and unloaded, the optical detector 12 also may be arranged at the same origin position P1 as the position where the module 10 is loaded and unloaded. Although the moving unit 13 moves the rotary table 90 with respect to the fixed optical detector 12, the rotary table 90 on the module installation unit 11 side may be stationary and the optical detector 12 moved.

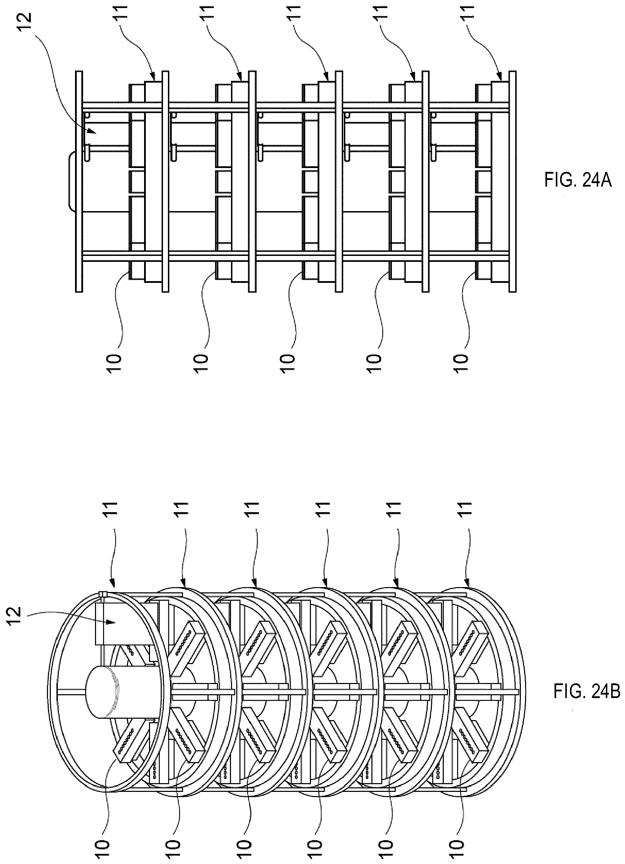

Although the module installation unit 11 is configured to arrange the plurality of modules 10 on the same plane at the same height in the circumferential direction of the circle in the present embodiment, a plurality of module installation units 11 may be provided so as to be stacked in the height direction as shown in FIG. 24. In the example of FIG. 24, a plurality of module installation units have a hierarchical structure in which they are stacked in the height direction, and an optical detector 12 is arranged in each layer. Therefore, one optical detector 12 can be shared by a plurality of modules 10 in each layer, and nucleic acid amplification for a large number of samples can be detected by a small number of optical detectors 12 in the example of FIG. 24.

### Second Embodiment

Although the moving unit 13 moves the optical detection unit 12 and the module installation unit 11 relatively in the circumferential direction R of the circle vi a the rotary table 90 in the first embodiment, the module installation unit 11 may install a plurality of modules 10 side by side in a linear direction and the the module installation unit 11 may be relatively moved in the linear direction. Hereinafter, this example will be described as a second embodiment. Note that the structures of the parts not particularly mentioned remain the same as those of the first embodiment.

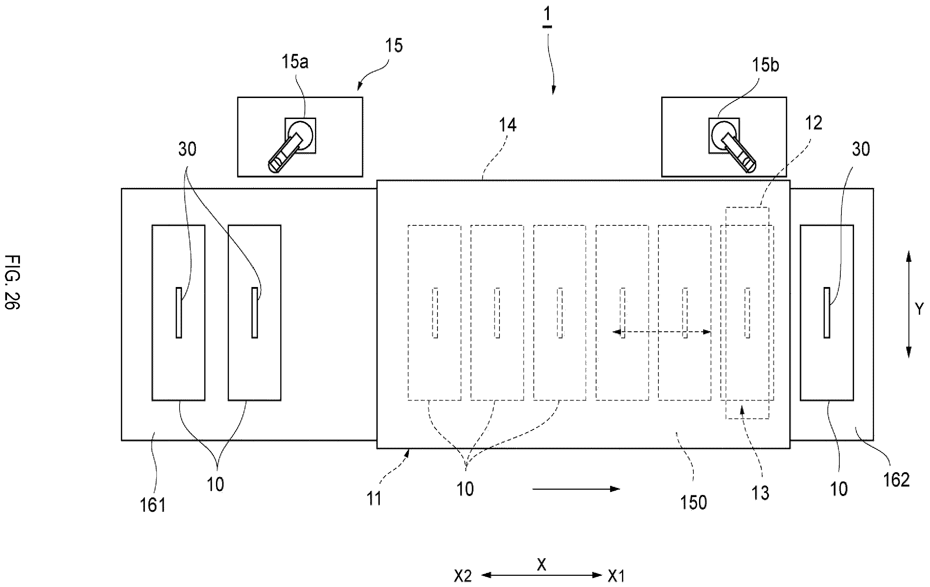

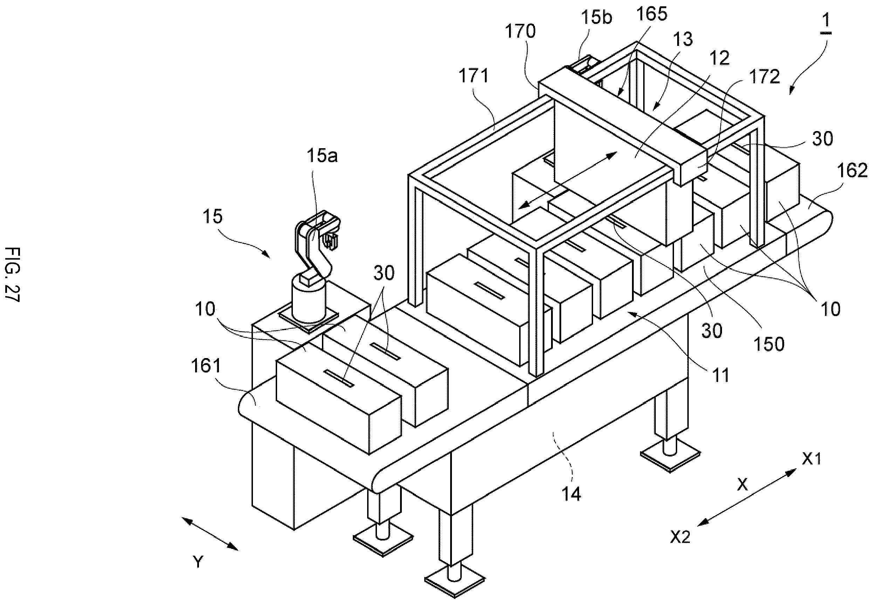

FIG. 25 is a perspective view of the nucleic acid detection device 1 according to the second embodiment, and FIG. 26 is a top view of the nucleic acid detection device 1. FIG. 27 is an descriptive diagram showing the structure of the nucleic acid detection device 1 in a state win which the housing of the main device is removed.

As shown in FIGS. 25 to 27, the module installation unit 11 has a conveyor 150 on which a plurality of modules 10 can be arranged side by side in a linear direction (X direction in the drawing). The conveyor 150 has a width larger than the length in the longitudinal direction of the module 10, and the module 10 can be placed in a state in which the longitudinal direction of the module 10 is oriented in the width direction (Y direction in the figure). The conveyor 150 has a horizontal upper surface, and a module 10 carried into the inlet side of the conveyor 150 by a drive unit (not shown) can be conveyed to the outlet side in the front direction in the X direction (X1 direction in the figure). The operation of the conveyor 150 is controlled by the CPU 14.

The device main body 80 includes, for example, a housing 160 that covers the conveyor 150, a loading stage 161 provided on the inlet side of the conveyor 150, and an unloading stage 162 provided on the outlet side of the conveyor 150.

As shown in FIG. 27, one optical detector 12 is provided above the conveyor 150. The moving unit 13 has a drive mechanism 165 that holds the optical detector 12 and moves it on the conveyor 150 in the X direction. The drive mechanism 165 may include, for example, a support part 170 that supports the optical detector 12, a slide rail 171 that slides the support part 170 in the X direction, a motor 172 that drives the support part 170 in the X direction, and the like. The operation of the drive mechanism 165 is controlled by the CPU 14.

The transport device 15 is, for example, configured by a loading robot 15a that carries in the module 10 on the loading stage 161 to the inlet side of the conveyor 150, and a unloading robot 15b that carries out the module 10 on the outlet side of the conveyor 150 onto the unloading stage 162.

Then, in the nucleic acid detection device 1, for example, a series of tubes 30 containing a sample are housed in a plurality of modules 10 placed on the loading stage 161 shown in FIGS. 25 and 26, and a reverse transcription process is performed by the temperature adjusting unit 50 in each module 10.

Next, the loading robot 15a places the plurality of modules 10 on the conveyor 150 in order. At this time, the conveyor 150 moves in the X1 direction. Then, as shown in FIG. 27, when the plurality of modules 10 are placed on the conveyor 150, the conveyor 150 stops.

Subsequently, the nucleic acid amplification process is started, and in each module 10, the temperature adjustment of the nucleic acid amplification cycle C is started by the temperature adjusting unit 50. At this time, as shown in FIG. 19, the starting points of the nucleic acid amplification cycle C of each module 10 are shifted from each other among the modules. Then, the optical detector 12 moves from one end of the conveyor 150, for example, from the outlet side end to the other end on the inlet side (in the X2 direction in the figure), and emits and receives light relative to each module 10 in order, and the amplified nucleic acid of the sample in each module 10 is detected. At this time, as shown in FIG. 20, the optical detector 12 is controlled to detect the amplified nucleic acid for each module 10 at a specific timing of the nucleic acid amplification cycle C, for example, when the sample is cooled to about 60°C.

When the optical detector 12 moves on the conveyor 150 to the inlet side end and returns to the outlet side end and again moves from the outlet side end to the inlet side end of the conveyor 150, such that the amplified nucleic acid of the sample is detected for each module 10 at a specific timing of the nucleic acid amplification cycle C of each module 10. As a result, the amplified nucleic acid of another module 10 is detected between the first detection and the second detection of the amplification nucleic acid for a particular module 10. Then, this is repeated a plurality of times, for example, 40 times, and the nucleic acid amplification process is completed.

The module 10 for which the nucleic acid amplification process has been completed is carried out to the unloading stage 162 from the outlet side of the conveyor 150 by the unloading robot 15b shown in FIGS. 25 and 26. When a space is created on the outlet side of the conveyor 150, the conveyor 150 moves in the X1 direction, and the next module 10 on the loading stage 161 is loaded into the space created on the inlet side of the conveyor 150 by the loading robot 15a.

According to the present embodiment, a plurality of modules 10 accommodating a series of tubes 30 are sequentially installed in the module installation unit 11, the temperature of each module 10 is adjusted for the nucleic acid amplification reaction, and the amplified nucleic acid of the sample can be detected by the optical detector 12 shared for each module 10. In this way a large number of samples can be processed without waiting time, and various examination requirements can be flexibly met. Further, the number of optical detectors 12 can be reduced, and as a result, the cost of the device can be reduced and the size of the device can be reduced. Since the optical detector 12 is shared, the periodic maintenance of the optical detector 12 can be reduced, and the burden on the user can be reduced.

The module installation unit 11 installs a plurality of modules 10 side by side in the linear direction X, and the moving unit 13 moves the optical detector 12 and the module installation unit 11 relatively in the linear direction X. In this way nucleic acid detection for a plurality of modules 10 can be suitably performed using the shared optical detector 12.

Although the temperature adjusting unit 50 is provided in the module 10 in the present embodiment, the temperature adjusting unit 50 also may be provided in the device main body 80, for example, the conveyor 150. Although there is one optical detector 12, there may be a plurality of optical detectors 12 insofar as the optical detectors 12 are shared by a plurality of modules 10.

Although preferred embodiments of the present invention have been described above with reference to the accompanying drawings, the present invention is not limited to such examples. It is understood that a person skilled in the art can devise various modifications or modifications within the scope of the ideas described in the claims, which naturally belong to the technical scope of the present invention.

For example, the nucleic acid detection device 1 described in the first and second embodiments need not include the transport device 15, and the user may load the module 10 into and out of the module installation unit 11. Although the nucleic acid detection device 1 has a function of performing reverse transcription processing and nucleic acid amplification processing, the function of performing reverse transcription processing also may be performed by another device. Other configurations of the nucleic acid detection device 1 are not limited to those of the first and second embodiments described above.

Although the plurality of modules 10 are heated and cooled at the same set temperatures in the nucleic acid detection device 1 described in the first and second embodiments, heating and cooling also may be performed for each module 10 at different set temperatures. Specifically, an example of a nucleic acid amplification cycle in which a sample is heated to 95°C and then cooled to 60°C is executed in each module has been described in the first and second embodiments, but the set temperature for heating and cooling may be changed for each module. For example, the temperature at which nucleic acid is annealed may differ depending on the PCR exam item and the type of exam reagent. Therefore, for example, a first module for measuring one PCR exam item may be set to heat to 95°C and cool to 60°C as set temperatures, and a second module for measuring another PCR exam item the temperature may be set at 80°C for heating and 50°C for cooling.

In the first and second embodiments, the example has been described in which the sample is heated to 45°C in the reverse transcription process and then the sample is maintained at a high temperature of 95°C until the nucleic acid amplification cycle is started. This temperature control is for causing a reverse transcription reaction, and is effective for amplifying RNA virus nucleic acid in a sample, for example. On the other hand, for viruses that have only DNA, it is necessary to cause denaturation instead of reverse transcription reaction. In order to cause denaturation of the sample, it is necessary to heat the sample to a high temperature state from the beginning instead of reverse transcription process. In other embodiments, the sample may be heated to 95°C instead of 45°C in the reverse transcription process to cause this denaturation.

### Industrial Applicability

The present invention is a nucleic acid detection device, a nucleic acid detection method, and module capable of processing a large number of samples with a short waiting time, flexibly responding to various examination requirements, and reducing the number of optical detectors.

### EXPLANATION OF REFERENCE NUMBERS

1 Nucleic acid detection device
10 Module
11 Module installation unit
12 optical detection unit
13 Moving unit
14 Device control unit
15 Transport device
30 tube
50 Temperature regulating unit

## Claims

1. A nucleic acid detection method comprising:
installing a plurality of modules capable of holding a container for containing a sample for a nucleic acid amplification reaction in a module installation unit;
adjusting a temperature of the sample so as to repeat a nucleic acid amplification cycle for each of the plurality of modules installed in the module installation unit;
moving the module installation unit relative to an optical detection unit shared by the plurality of modules, and positioning each of the plurality of modules at a position where the optical detection unit configured to detect an amplified nucleic acid of the sample; and
detecting the amplified nucleic acid of the sample for each of the plurality of modules by the optical detection unit.

2. The nucleic acid detection method according to claim 1, wherein
the moving comprises sequentially moving the module installation unit relative to the optical detection unit, and
the positioning comprises sequentially positioning each of the plurality of modules at the position.

3. The nucleic acid detection method according to claim 1 or 2, wherein
the optical detection unit is configured to detect the amplified nucleic acid for each of the plurality of modules at a specific timing of the nucleic acid amplification cycle,
the detecting is performed a predetermined number of times according to the repeated nucleic acid amplification cycle, and
the amplified nucleic acid is detected for another module among the plurality of modules between a first detection of the amplified nucleic acid and a second detection of the amplified nucleic acid for a predetermined module among the plurality of modules.

4. The nucleic acid detection method according to claim 3, wherein
the adjusting comprises adjusting the temperature of the sample so that a starting point of the repeated nucleic acid amplification cycle shifts in each of the plurality of modules.

5. The nucleic acid detection method according to any one of claims 1 to 4, wherein
the adjusting is performed by a temperature adjusting unit provided in each of the plurality of modules.

6. The nucleic acid detection method according to any one of claims 1 to 5, wherein
the adjusting is performed by a temperature adjusting unit provided in the module installation unit.

7. The nucleic acid detection method according to any one of claims 1 to 6, wherein
the adjusting comprises allowing each of the plurality of modules to raise and lower the temperature at different set temperatures.

8. The nucleic acid detection method according to any one of claims 1 to 7, further comprising:
installing each of the plurality of modules in the module installation unit by a transfer device; and/or
removing each of the plurality of modules installed in the module installation unit by the transfer device.

9. The nucleic acid detection method according to any one of claims 1 to 8, wherein
the module installation unit is configured to install each of the plurality of modules side by side in a circumferential direction of a circle; and
the moving comprises moving the optical detection unit and the module installation unit relatively in the circumferential direction of the circle.

10. The nucleic acid detection method according to any one of claims 1 to 9, wherein
the module installation unit is configured to install each of the plurality of modules side by side in a linear direction; and
the moving comprises moving the optical detection unit and the module installation unit relatively in the linear direction.

11. The nucleic acid detection method according to claim any one of claims 1 to 10, further comprising:
carrying out a reverse transcription reaction of the sample in the container before each of the plurality of modules is arranged in the module installation unit.

12. A nucleic acid detection device comprising:
a plurality of modules configured to hold a container for containing a sample;
a module installation unit configured to install the plurality of modules;
an optical detection unit configured to detect an amplified nucleic acid of the sample contained in the container, and to be shared by the plurality of modules installed in the module installation unit; and
a moving unit configured to relatively move the optical detection unit and the module installation unit so that the optical detection unit detects the amplified nucleic acid of the sample in the container for each of the plurality of modules installed in the module installation unit,
wherein at least one of the plurality of the modules and the module installation unit includes a temperature adjusting unit for amplifying an nucleic acid of the sample contained in the container.

13. A module comprising:
a main body;
an accommodating unit provided on a surface of the main body and capable of accommodating a container for containing a sample in which a nucleic acid amplification is to be performed; and
a temperature adjusting unit, provided in the main body, for adjusting a temperature of the sample of the container accommodated in the accommodating unit;
wherein the temperature adjusting unit comprises a heating source, a temperature controller, and a temperature sensor.

14. The module according to claim 13, further comprising;
a communication unit configured to communicate with an outside.

15. The module according to claim 14, wherein the communication unit is configured to communicate via a wireless communication.
